# EUROPEAN PATENT SPECIFICATION

(11) **EP 1 649 831 B1**
(45) Date of publication and mention of the grant of the patent: **22.04.2009**
(21) Application number: 04745341.0
(22) Date of filing: 26.05.2004
(51) Int. Cl.: A61F 2/16

(54) **INJECTOR FOR INTRAOCULAR LENS**
INJEKTIONSGERÄT FÜR INTRAOKULARLINSE
INJECTEUR POUR LENTILLE INTRAOCULAIRE

(30) Priority: 27.05.2003 JP 2003149215
(43) Date of publication of application: 26.04.2006
(73) Proprietor: HOYA CORPORATION, 161-8525 Tokyo (JP)
(72) Inventor: FUTAMURA,Hideyuki HOYA CORPORATION, Shinjuku-ku, Tokyo (JP)
(74) Representative: Beetz & Partner
(86) International application number: PCT/JP2004/007189
(87) International publication number: WO 2004/105648

(56) References cited:
- JP-A- 7 047 091
- JP-A- 7 328 045
- JP-Y2- 3 050 894
- US-A- 6 162 229
- US-A1- 2002 133 167

## Description

### Technical Field

The present invention relates to an injector which is used at the time of inserting an intraocular lens into a capsula lentis.

### Background Art

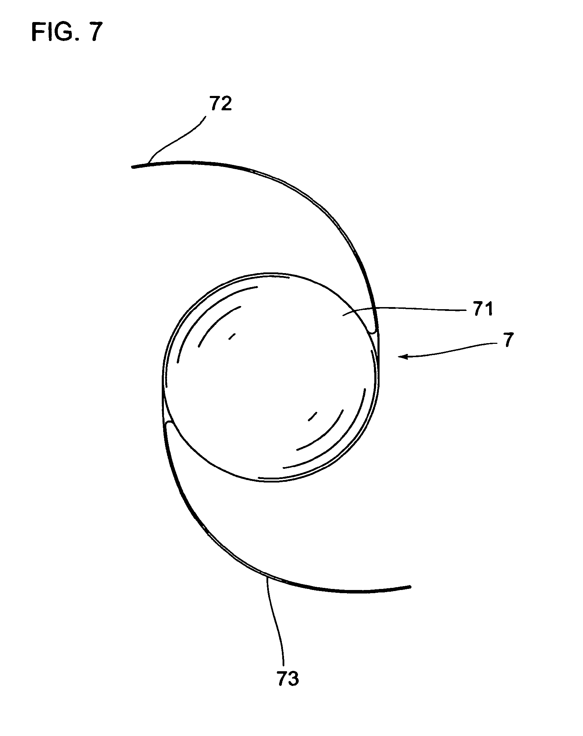

There is a method of implanting an intraocular lens 7 which has, as illustrated in FIG. 7, an optical part 71, and two loops 72 and 73 provided on opposing portions of the optical part 71 and supporting the optical part 71 from an incision formed in an eyeball instead of a crystalline lens which has lost its function due to cataract, thereby recovering a vision (see, for example, Patent Literature 1).

From the standpoint of hastening the recovery of a patient, it is desired that the incision is as small as possible. Because of this demand, a soft material, such as a silicon resin, an acrylate resin, or a hydrogel, is recently used as the material of the intraocular lens 7, and a surgery of inserting the intraocular lens from a small incision with that lens rolled or folded is performed.

As the method of the surgery, there is a method of folding the intraocular lens 7 front side out by a forceps, inserting the lens in such a way that the loop 72 inserted in first becomes parallel to an eye of a patient, after which the intraocular lens 7 is released with the loop 72 kept in parallel, while being rotated clockwise as viewed from an operator in such a way that the back side of the intraocular lens 7 faces the vitreous-body side.

According to the method, however, handling the forceps is difficult, and there are risks such as dropping the intraocular lens 7 and enlarging the incision, which brings about a problem such that the surgery largely depends on the skill of the operator.

Recently, as means of solving such a problem, there is an injector comprising a cartridge in which an intraocular lens 7 can be loaded with the front side thereof folded inwardly, a cylindrical injector main body with mount means on which the cartridge is detachably mounted, and ejection means which is fitted into the injector main body and can eject the intraocular lens 7, loaded in the cartridge, out of the cartridge (see, for example, Patent Literature 2).
Patent Literature 1: Japanese Unexamined Patent Publication No. H7-255757 (p. 2, FIG. 3)
Patent Literature 2: Japanese Unexamined Patent Publication No. 2003-70830 (p. 3, FIG. 1)

With the injector in use, because the loop 72 on the ejection side of the intraocular lens 7 loaded in the cartridge is tilted, if the intraocular lens 7 is inserted as it is, the loop 72 may harm a cornea or the like. To prevent this, the lens has only to be inserted in such a way that the loop 72 on the ejection side of the intraocular lens 72 loaded in the cartridge is in parallel to an eye of a patient. Since the loop 72 is very small, however, there is a problem such that it is difficult to figure out a location where the loop 72 becomes parallel.

In a case where the lens is inserted in such a way that the loop 72 becomes parallel to the surface of the eye, the intraocular lens 7 may be turned upside down if the intraocular lens 7 is released as it is, making it necessary to rotate the lens counterclockwise as viewed from an operator so that the front side of the intraocular lens 7 faces the surface of the eye. As this rotation direction is opposite to the direction in the case where the forceps is used, however, there is a problem such that the operator is liable to mishandle the work. To resolve this, a cartridge in which the intraocular lens 7 can be loaded front side out may be used. Even in this case, however, there are problems such that the operator would wonder in which direction the injector should be rotated, and would have a difficulty in determining how much it should be rotated.

US 2002/01331167 A1 discloses an apparatus for inserting an intraocular lens having a loop into an eye, which has a hollow tube defining a hollow space through which an intraocular lens is passed and an outlet through which the intraocular lens is inserted into the eye.

The present invention has been made in view of the circumstances, and it is an object of the invention to provide an injector which enables an operator to safely insert an intraocular lens without a mishandling.

To achieve the object, an injector as set forth in claim 1 is provided.

Accordingly, as it is possible to easily figure out the orientation of the intraocular lens loaded in the cartridge at the time the intraocular lens is inserted, a cornea or the like can be prevented from being harmed by the loop of the intraocular lens.

The injector as set forth in claim 2 is characterized in that the insertion-start-position indicating means is formed at a location which inclines with respect to the insertion-complete-position indicating means by an angle greater than 0° and less than 90°with a straight line, as a rotation axis, along which the intraocular lens moves when pushed out.

Accordingly, as it is possible to easily figure out the orientation of the intraocular lens loaded in the cartridge at the time the intraocular lens is inserted, a cornea or the like can be prevented from being harmed by the loop of the intraocular lens.

The injector as set forth in claim 3 is characterized in that the insertion-start-position indicating means is formed at a location which inclines with respect to said insertion-complete-position indicating means by an angle greater than 0° and less than 45° with a straight line, as a rotation axis, along which said intraocular lens moves when inserted.

Accordingly, as it is possible to easily figure out the orientation of the intraocular lens loaded in the cartridge at the time the intraocular lens is inserted, a cornea or the like can be prevented from being harmed by the loop of the intraocular lens.

The injector as set forth in claim 4 is characterized in that the insertion-start-position indicating means is formed at a location inclined at a clockwise angle with respect to the insertion-complete-position indicating means with a straight line, as a rotation axis, along which the intraocular lens moves when inserted in a case where the intraocular lens is inserted from a near side toward a far side.

Accordingly, also in the injector which folds the intraocular lens front side out and rotates the lens in the same direction as that in the case of using a forceps, it is possible to easily figure out an angle at which the ejection-side loop of the intraocular lens loaded in the cartridge becomes in parallel to the surface of the eye. It is also possible to easily figure out the direction of rotating the cartridge when the intraocular lens is ejected into the eye, thus preventing the inserted intraocular lens from turning upside down.

The injector according to claim 5 is characterized by comprising rotation-direction indicating means for indicating a direction in which the cartridge is rotated when the intraocular lens is inserted in the eye.

This makes it possible to easily figure out the direction of the rotation and the amount of the rotation, so that the inserted intraocular lens does not turn upside down.

### Brief Description of Drawing

[FIG. 1] A perspective view illustrating an injector according to a first embodiment of the invention.
[FIG. 2] A front view illustrating ejection means of the invention.
[FIG. 3] A schematic perspective view illustrating mount means of the invention.
[FIG. 4] A perspective view illustrating a cartridge.
[FIG. 5] An explanatory diagram illustrating how to use the injector of the invention.
[FIG. 6] A perspective diagram illustrating an injector according to a second embodiment of the invention.
[FIG. 7] A front view of an intraocular lens as viewed from the front side.

### Best Mode for Carrying Out the Invention

Embodiments of the invention will now be explained with reference to the accompanying drawings.

A first embodiment of the invention is an injector provided with insertion-start-position indicating means.

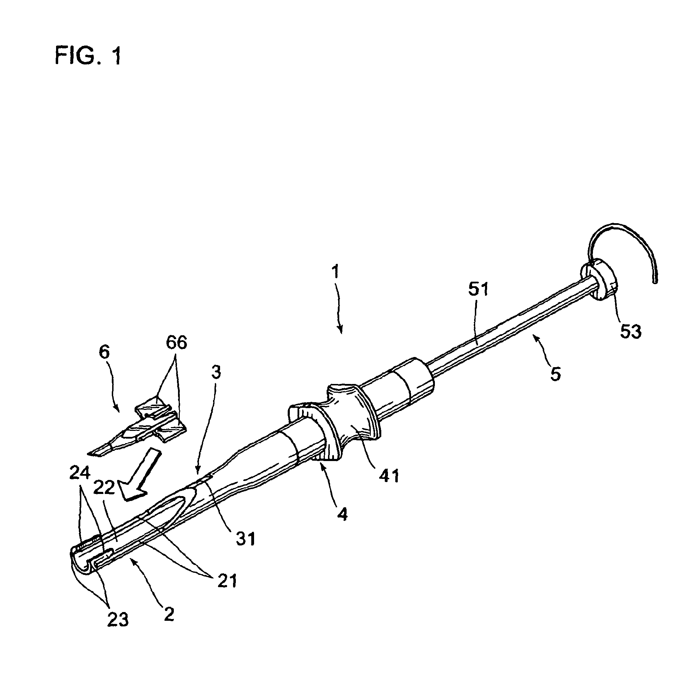

As illustrated in FIG. 1, an injector 1 comprises an injector main body 4 having insertion-complete-position indicating means 2 on which a cartridge 6 is detachably mounted, and which indicates a position as a measure of completing insertion of an intraocular lens and insertion-start-position indicating means 3 which indicates a position as a measure of starting the insertion of the intraocular lens 7, and ejection means 5 which can eject the intraocular lens 7, loaded in the cartridge 6, out of the cartridge 6. Various materials can be used for the injector 1 unless they cause a trouble in the insertion of the intraocular lens 7 into an eye; for example, metals, such as stainless steel and titanium, can be used.

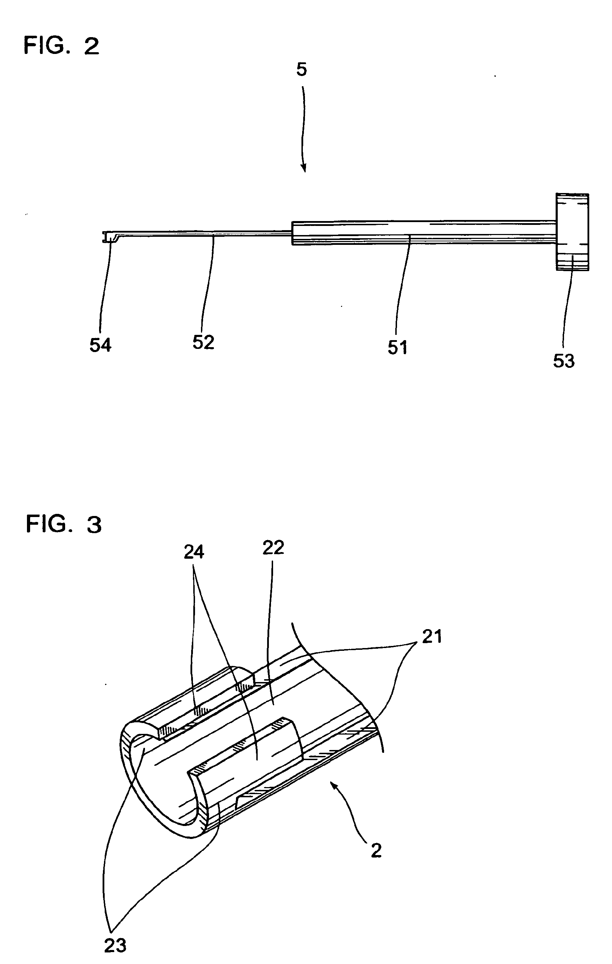

As illustrated in FIG. 2, the ejection means 5 comprises a base 51 which is fitted into the injector main body 4, a pushrod 52 which is formed on one end side of the base 51, and pushes out the intraocular lens 7 loaded in the cartridge 6, and a latch portion 53 which is so formed on the other end side of the base 51 as to be larger than the diameter of the injector main body 4. Formed on the leading end of the pushrod 52 is a hoe-like shaped push-out portion 54 which, in abutment on the side face of the folded intraocular lens 7, can push the lens out.

The injector main body 4 is formed like a cylinder so as to have a fitting channel inside into which the base 51 of the ejection means 5 can be fitted from one end side. Support means 41 by which the fingers or the like of an operator are fixed at the time of the insertion of the intraocular lens 7 is fixed on the exterior surface of the injector main body 4 by fixing means like a screw. The other end side of the injector main body 4 is provided with the insertion-complete-position indicating means 2 on which the cartridge 6 is detachably mounted, and which indicates the measure of completing the insertion of the intraocular lens.

As illustrated in FIG. 3, the insertion-complete-position indicating means 2 is formed with a guide section 22 hemi-arcuate in cross section which has two guide channels 21 for guiding a wing portion 66 of the cartridge and protrudes from the injector main body 4, and the two guide channels 21 indicate the measure of completing the insertion of the intraocular lens 7. Formed on the leading-end side of the guide section 22 are a front-side latch piece 23 which protrudes upward and can latch the front side of the wing portion 66, and a top-side latch piece 24 which protrudes toward the injector main body 4 from the upper part of the front-side latch piece 23, and can latch the top side of the wing portion 66. The insertion-complete-position indicating means 2 is not limited to that as long as the cartridge 6 can be mounted thereon, and may take other structures.

As illustrated in FIG. 1, the insertion-start-position indicating means 3 is constituted by a flat-surface portion 31 which is notched in the side of the injector main body 4, and indicates the position where a loop 72 on the ejection side at the time of starting the insertion of the intraocular lens 7 becomes horizontal in a case where the orientation of the insertion-start-position indicating means 3 is set horizontal. For example, in a case where the cartridge 6 in which the intraocular lens 7 is loaded while being folded front side out is used, the flat-surface portion 31 may be formed at a location inclined by an angle greater than 0° and less than 90° with respect to the insertion-complete-position indicating means 2 with a straight line, as the rotation axis, along which the intraocular lens 7 moves when pushed out, and it is preferable that the flat-surface portion should be formed at a location by an angle greater than 0° and less than 45°. Accordingly, arranging the flat-surface portion 31 in parallel with an eye can make the loop 72 on the ejection side parallel to the surface of the eye at the time of starting the insertion.

The insertion-start-position indicating means 3 is not limited to one formed in a planar shape by notching the curved surface of the injector main body 4, and, for example, other methods like coloring may be applicable.

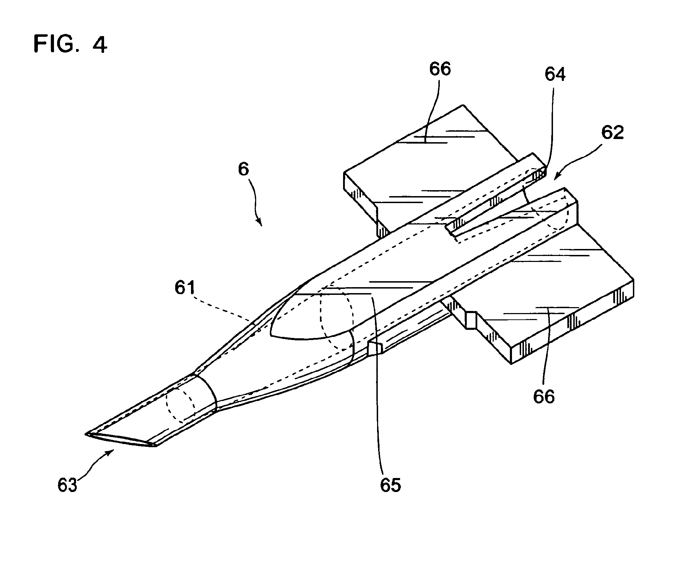

The cartridge 6 which is mounted on the injector 1 comprises, as illustrated in, for example, FIG. 4, a cartridge main body 65 which has an introduction tube 61, an inlet portion 62, an outlet portion 63, and insertion grooves 64, and the wing portion 66 which is so formed as to protrude from both sides of the cartridge main body 65 and be attachable to and detachable from the insertion-complete-position indicating means 2.

The inlet portion 62 is for loading the folded intraocular lens 7, and its upper and bottom parts are provided with the insertion grooves 64. The insertion grooves 64 are for loading the intraocular lens 7 held by a forceps, and the insertion of the forceps into the insertion grooves 64 facilitates the insertion of the folded intraocular lens 7 into the inlet portion 62. The interior of the introduction tube 61 is formed like a tubular shape through which the folded and loaded intraocular lens 7 can pass from the inlet portion 62 toward the outlet portion 63.

Given that the lengthwise direction of the introduction tube 61 is a longitudinal axis, the outlet portion 63 is formed angled to the longitudinal axis, i.e., the cut surface is oblique. The leading end part of the outlet portion is so formed as to incline with respect to the longitudinal axis at the same angle as that of the insertion-start-position indicating means 3 when the cartridge is mounted on the injector. This can facilitate insertion of the outlet portion 63 into the incision of the eye.

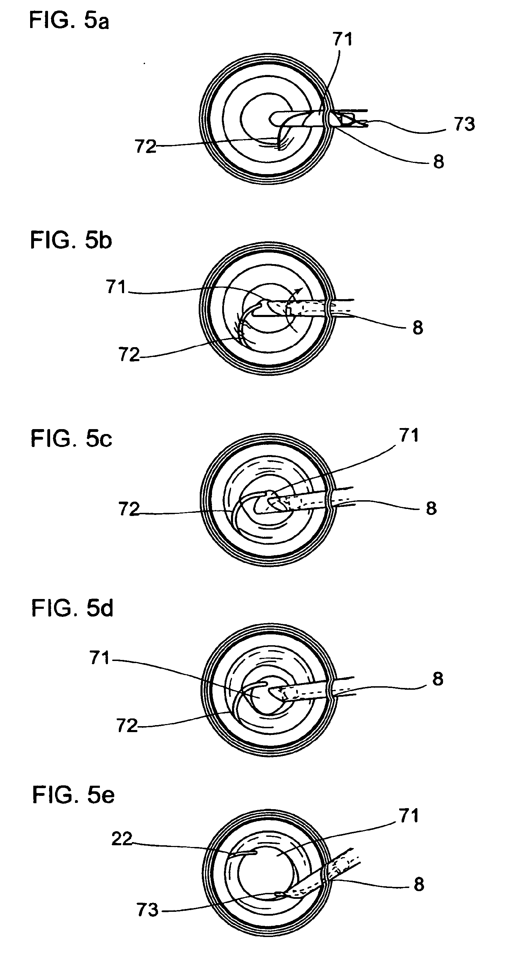

Next, how to use the injector 1 will be explained with reference to FIG. 5.

First, the intraocular lens 7 is folded and picked up by a forceps or the like in such a way that the front of that lens faces outward, and is inserted into the inlet portion 62 of the cartridge 6 in such a way that the loop 72 on the ejection side comes above the other loop 73. Next, the bottom face of the wing portion 66 of the cartridge 6 is made to abut on the guide paths 21 of the insertion-complete-position indicating means 2, slid toward the front-side latch piece 23 in that state, and the wing portion 66 is sandwiched and supported between the top-side latch piece 24 and the guide channels 21. Subsequently, the push-out portion 54 of the ejection means 5 is made to abut on the side of an optical part 71 of the intraocular lens 7 to push out the lens until the leading end of the loop 72 on the ejection side reaches the outlet portion 63. This will finish preparation of the injector 1.

Next, with the flat-surface portion 31 of the insertion-start-position indicating means 2 so supported as to be parallel to the surface of the eye, the ejection nozzle 63 of the cartridge 6 is inserted into an incision 8 of the eyeball, and the loop 72 on the ejection side is pushed out into the eye. Accordingly, the loop 72 can be inserted while being kept in parallel to the eye (see FIG. 5(a)).

After that, an operator gradually pushes out the intraocular lens 7 into the eye though the ejection means 5 while keeping the loop 72 on the ejection side in parallel to the surface of the eye (see FIG. 5(b) or (d)), and releases the optical part (see FIG. 5(e)). That is, the injector 1 is rotated clockwise to the direction of insertion, at which time the insertion-complete-position indicating means 2 becomes the measure of the insertion-complete position, and it is preferable that the injector should be rotated beyond the insertion-complete position to safely insert the intraocular lens 7 without turning around that lens in the eye. Because the operator has only to rotate the injector 1 in a direction in which the insertion-complete-position indicating means 2 becomes horizontal, it is possible to safely insert the intraocular lens 7 without making the rotation in the wrong direction.

Finally, the injector 1 is so operated as to set the rear loop 73 in a capsula, and the outlet portion 63 is pulled off from the incision, thereby finishing the operation.

A second embodiment of the invention is an injector provided with rotation-direction indicating means.

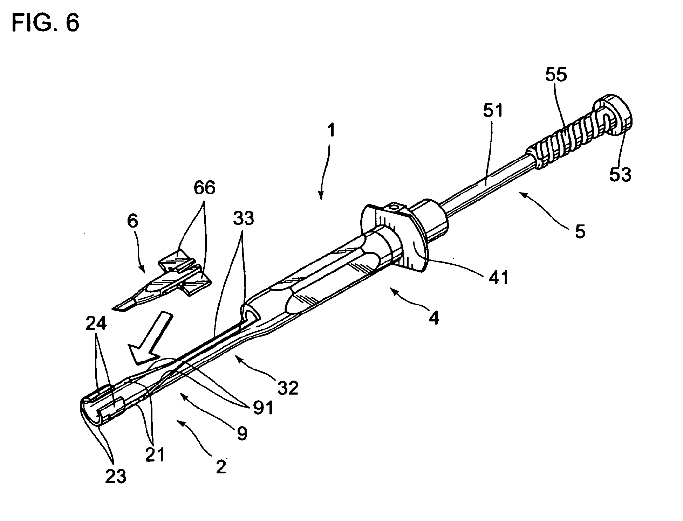

As illustrated in FIG. 6, insertion-start-position indicating means 32 is formed in a hemi-arcuate shape in cross section by cutting the injector main body 4 in half at an arbitrary width, and is formed in such a way that the loop 72 on the ejection side becomes parallel to the surface of an eye when two insertion-position indication portions 33 are arranged in parallel to the surface of the eye.

Rotation-direction indicating means 9 is so formed in a hemi-arcuate shape in cross section as to have a rotation-direction indication channels 91 which connect the insertion-position indication portions 33 of the insertion-start-position indicating means 32 to the guide channels 21 of the insertion-complete-position indicating means 2, and is so formed as to turn the insertion-complete-position indicating means 2 counterclockwise from the insertion-start-position indicating means 32 with respect to the direction of inserting the intraocular lens 7 into the eye. In this case, if the orientation of inserting the intraocular lens 7 is toward the far side from the near side with respect to the direction of inserting the intraocular lens 7 into the eye, the insertion-complete-position indicating means 2 may be formed at a location counterclockwise from the insertion-start-position indicating means 32 by an angle greater than 0° and less than 90°, and preferably formed at a location counterclockwise from the insertion-start-position indicating means 32 by an angle greater than 0° and less than 45°. As the rotation-direction indicating means 9 is structured in this manner, an operator has only to rotate the injector 1 along the rotation-direction indication channels 91 after ejecting the loop 72 on the ejection side in the eye, and can therefore easily figure out the direction of rotating the injector 1, and surely fit the inserted intraocular lens 7 without turning it upside down.

In the ejection means 5, a quadruple thread portion 55 is provided between the base 51 and the latch portion 53, and an engagement portion (not illustrated) which can engage with a groove 56 of the quadruple thread portion is provided on the interior surface of the injector main body 4. Accordingly, the ejection means can be pushed forward while being rotated with the groove 56 of the quadruple thread 55 engaged with the engagement portion, and fitting the base 51, pushrod 52, and the push-out portion 54 of the ejection means 5 can be slacked, and the intraocular lens 7 can be ejected safely.

In FIG. 5, the same portions as those of the first embodiment are denoted by the same reference numerals to omit their explanations.

In the embodiments, although explanations have been given of the case where the injector 1 uses the cartridge 6 in which the intraocular lens 7 folded front side out is loaded, the embodiments are not restrictive, and needless to say, the invention is also applicable to a case where the injector 1 uses the cartridge 6 in which the intraocular lens 7 folded front side in is loaded. In this case, for example, the insertion-complete-position indicating means 2 may be formed at a location rotated clockwise from the insertion-start-position indicating means 3 by an angle greater than 0° and less than 90° if the orientation of the intraocular lens 7 is toward the far side from the near side with the straight line, as a rotation axis, along which the intraocular lens 7 moves at the time of the insertion, and the insertion-complete-position indicating means 2 should preferably be formed at a location inclined clockwise from the insertion-start-position indicating means 3 by an angle greater than 0° and less than 45°.

## Claims

1. An injector for inserting an intraocular lens (7) which has a loop (72) loaded in a cartridge (6) into an eye by pushing out said lens (7)
**characterized in that** the injector is configured for pushing out said lens while rotating said lens and comprises insertion-start-position indicating means (3; 32) for indicating a rotational position of the injector at a time of starting inserting said intraocular lens (7), and insertion-complete-position indicating means (2) for indicating a rotational position of the injector at a time of completing the insertion of said intraocular lens (7).

2. The injector according to claim 1, wherein said insertion-start-position indicating means (3; 32) is formed at a location which inclines with respect to said insertion-complete-position indicating means (2) by an angle greater than 0° and less than 90°with a straight line, as a rotation axis, along which said intraocular lens (7) moves when pushed out.

3. The injector according to claim 1, wherein said insertion-start-position indicating means (3; 32) is formed at a location which inclines with respect to said insertion-complete-position indicating means (2) by an angle greater than 0° and less than 45° with a straight line, as a rotation axis, along which said intraocular lens (7) moves when inserted.

4. The injector according to claim 1, wherein said insertion-start-position indicating means (3; 32) is formed at a location inclined at a clockwise angle with respect to said insertion-complete-position indicating means (2) with a straight line, as a rotation axis, along which said intraocular lens moves when inserted in a case where said intraocular lens (7) is inserted from a near side toward a far side.

5. The injector according to claim 1, comprising rotation-direction indicating means (9) for indicating a direction in which said cartridge (6) is rotated when said intraocular lens (7) is inserted in the eye.

## Patentansprüche

1. Injektionsgerät zum Einsetzen einer in eine Kartusche (6) geladenen Intraokularlinse (7), die eine Schlaufe (72) aufweist, in ein Auge durch Herausschieben der Linse (7),
**dadurch gekennzeichnet, dass** das Injektionsgerät zum Herausschieben der Linse beim Drehen der Linse konfiguriert ist und eine Einsetzungsstartpositions-Anzeigeeinrichtung (3; 32) zum Anzeigen der Drehposition des Injektionsgeräts zum Zeitpunkt des Beginns des Einsetzens der Intraokularlinse (7) und eine Einsetzungsabschlusspositions-Anzeigeeinrichtung (2) zum Anzeigen der Drehposition des Injektionsgeräts zum Zeitpunkt des Abschlusses des Einsetzens der Intraokularlinse (7) umfasst.

2. Injektionsgerät nach Anspruch 1, wobei die Einsetzungsstartpositions-Anzeigeeinrichtung (3; 32) an einer Stelle ausgebildet ist, die sich bezüglich der Einsetzungsabschlusspositions-Anzeigeeinrichtung (2) um einen Winkel von mehr als 0° und weniger als 90° mit einer Geraden als Drehachse neigt, an der sich die Intraokularlinse (7) beim Herausschieben entlang bewegt.

3. Injektionsgerät nach Anspruch 1, wobei die Einsetzungsstartpositions-Anzeigeeinrichtung (3; 32) an einer Stelle ausgebildet ist, die sich bezüglich der Einsetzungsabschlusspositions-Anzeigeeinrichtung (2) um einen Winkel von mehr als 0° und weniger als 45° mit einer Geraden als Drehachse neigt, an der sich die Intraokularlinse (7) beim Einsetzen entlang bewegt.

4. Injektionsgerät nach Anspruch 1, wobei die Einsetzungsstartpositions-Anzeigeeinrichtung (3; 32) an einer Stelle ausgebildet ist, die in einem Winkel im Uhrzeigersinn bezüglich der Einsetzungsabschlusspositions-Anzeigeeinrichtung (2) mit einer Geraden als Drehachse geneigt ist, an der sich die Intraokularlinse beim Einsetzen in einem Fall entlang bewegt, in dem die Intraokularlinse (7) von einer nahen Seite zu einer entfernten Seite hin eingesetzt wird.

5. Injektionsgerät nach Anspruch 1, mit einer Drehrichtungs-Anzeigeeinrichtung (9) zum Anzeigen einer Richtung, in der die Kartusche (6) gedreht wird, wenn die Intraokularlinse (7) in das Auge eingesetzt wird.

## Revendications

1. Injecteur permettant d'insérer dans un oeil une lentille intra-oculaire (7) qui possède une boucle (72) chargée dans une cartouche (6) en poussant ladite lentille (7) vers l'extérieur
**caractérisé en ce que** l'injecteur est configuré pour pousser ladite lentille vers l'extérieur tout en faisant tourner ladite lentille et comprend un moyen d'indication de la position de début d'insertion (3 ; 32) permettant d'indiquer une position de rotation de l'injecteur au moment du début de l'insertion de ladite lentille intra-oculaire (7), et
un moyen d'indication de la position de fin d'insertion (2) permettant d'indiquer une position de rotation de l'injecteur au moment de la fin de l'insertion de ladite lentille intra-oculaire (7).

2. L'injecteur selon la revendication 1, dans lequel ledit moyen d'indication de la position de début d'insertion (3 ; 32) est formé dans un endroit qui est incliné par rapport audit moyen d'indication de la position de fin d'insertion (2) formant un angle supérieur à 0° et inférieur à 90° avec une ligne droite, faisant office d'axe de rotation, le long de laquelle ladite lentille intra-oculaire (7) se déplace quand elle est poussée vers l'extérieur.

3. L'injecteur selon la revendication 1, dans lequel ledit moyen d'indication de la position de début d'insertion (3 ; 32) est formé dans un endroit qui est incliné par rapport audit moyen d'indication de la position de fin d'insertion (2) formant un angle supérieur à 0° et inférieur à 45° avec une ligne droite, faisant office d'axe de rotation, le long de laquelle ladite lentille intra-oculaire (7) se déplace quand elle est insérée.

4. L'injecteur selon la revendication 1, dans lequel ledit moyen d'indication de la position de début d'insertion (3 ; 32) est formé dans un endroit qui est incliné formant un angle dans le sens des aiguilles d'une montre par rapport audit moyen d'indication de la position de fin d'insertion (2) avec une ligne droite, faisant office d'axe de rotation, le long de laquelle la lentille intra-oculaire se déplace quand elle est insérée dans le cas où ladite lentille intra-oculaire (7) est insérée à partir d'un côté proche vers un côté éloigné.

5. L'injecteur selon la revendication 1, comprenant un moyen d'indication de la direction de rotation (9) permettant d'indiquer une direction dans laquelle ladite cartouche (6) tourne quand ladite lentille intra-oculaire (7) est insérée dans l'oeil.
